# EUROPÄISCHE PATENTANMELDUNG

(11) **EP 4 755 437 A2**
(43) Veröffentlichungstag der Anmeldung: **10.06.2026**
(21) Anmeldenummer: 26174578.0
(22) Anmeldetag: 23.09.2010
(51) Int. Cl.: A61M 39/10

(54) **VERRIEGELBARE SCHNELLKUPPLUNG**

(30) Priorität: 30.09.2009 DE 102009047844
(62) Teilanmeldung aus: 21160539.9
(71) Anmelder: Abiomed Europe GmbH, 52074 Aachen (DE)
(72) Erfinder: SIESS, Thorsten, 52074 Aachen (DE); SPANIER, Gerd, 52074 Aachen (DE)
(74) Vertreter: Klunker IP Patentanwälte PartG mbB

(57) **Zusammenfassung**

Eine einfach verriegelbare Schnellkupplung (10) zum lösbaren Verbinden eines Schlauchs (12) mit einem implantierbaren Apparat weist ein mit dem Schlauch (12) verbindbares, erstes Ansatzstück (14) und ein mit dem Apparat verbindbares zweites Ansatzstück (16) auf, wobei die Ansatzstücke (14, 16) im verriegelten Zustand gegeneinander verdrehbar sind.

## Beschreibung

Die Erfindung betrifft eine verriegelbare Schnellkupplung zum lösbaren Verbinden eines Schlauchs mit einem implantierbaren Apparat.

Bei implantierbaren Apparaten handelt es sich typischerweise um medizinische Hilfsapparate, wie zum Beispiel Blutpumpen, die in den Körper eines Patienten implantiert werden und mit einem eine Flüssigkeit fördernden Schlauch zu verbinden sind. Im Fall einer Blutpumpe ist ein Ende des Schlauchs typischerweise mit einer Arterie und das andere Ende mit der Blutpumpe zu verbinden. Hierbei besteht die Schwierigkeit, dass beim Verbinden des Schlauchs mit dem implantierten Apparat durch die entstehenden Kräfte die Verbindung zwischen Schlauch und Arterie beschädigt werden könnte.

Aus U.S. 7,273,446 B2 ist eine Blutpumpe bekannt, die in Bezug auf die Pulsation des Herzens antizyklisch arbeitet. Bei einer solchen gegenpulsatilen, CPD ("counter pulsation device") genannten Blutpumpe wird der das Blut fördernde Schlauch mit der Subclavia fest vernäht und die Pumpe in die rechte Herzschrittmachertasche im Brustbereich des Patienten implantiert. Die Pumpe saugt die während der Systole vom Herzen ausgeworfene Blutmenge an und reduziert dabei die vom Herzmuskel aufzubringende Kraft. Nach Schließen der Aortenklappe pumpt das CPD die zuvor aufgenommene Blutmenge während der Diastole in die Arterie. Bei der Implantation eines CPD ist die Pumpe mit dem mit der Subclavia vernähten Schlauch zu verbinden. Hierbei überträgt der Schlauch Torsions- und andere Kräfte auf die Verbindungsnaht mit der Subclavia. Das Verbinden von Schlauch und Pumpe muss daher mit großer Vorsicht erfolgen, um nicht die Verbindungsnaht an der Subclavia zu beschädigen. Zudem muss die Verbindung luftfrei und ohne sichtbare Übergangskanten erfolgen.

Der Erfindung liegt die Aufgabe zugrunde, das Verbinden eines Schlauchs mit einem implantierbaren Apparat zu erleichtern.

Die erfindungsgemäße Lösung ist definiert durch die Merkmale von Anspruch 1. Demnach wird eine verriegelbare Schnellkupplung zum lösbaren Verbinden eines Schlauchs mit einem implantierbaren Apparat vorgeschlagen. Die Schnellkupplung weist ein mit dem Schlauch verbindbares erstes Ansatzstück und ein mit dem Apparat verbindbares zweites Ansatzstück auf. Die beiden Ansatzstücke sind lösbar miteinander verriegelbar und im verriegelten Zustand gegeneinander verdrehbar.

Die beiden Ansatzstücke können auf einfache Weise miteinander verbunden werden, zum Beispiel nachdem der Apparat in den Patienten implantiert und der Schlauch mit einer Arterie des Patienten vernäht wurde. Beim Verbinden der beiden Ansatzstücke sind geringere Kräfte als bisher aufzubringen. Im Falle eines CPD werden geringere Kräfte auf den Schlauch ausgeübt und die Gefahr einer Beschädigung der Verbindungsnaht mit der Subclavia ist vermindert. Insbesondere kann die Verbindung zwischen dem ersten Ansatzstück und dem Schlauch bereits vor der Auslieferung der Schnellkupplung erfolgen, so dass von dem Operateur nur noch die beiden Ansatzstücke miteinander zu verbinden sind, um den Schlauch an den Apparat anzuschließen. Dadurch, dass die beiden Ansatzstücke im verriegelten Zustand gegeneinander verdrehbar sind, ist die Gefahr einer Torsion des Schlauchs beim Herstellen der Verbindung mit dem Apparat vermindert oder kann mindestens unmittelbar nach der Verbindung korrigiert werden. Die Schnellkupplung ermöglicht zudem eine besonders kleine Bauform mit geringem zusätzlichen Durchmesser, einer geringen Wandstärke und einer kurzen Bauform. Die geringe Größe der Schnellkupplung ist von besonderer Bedeutung, um auf begrenzten Raum während der Implantation die Verbindung des Schlauchs mit dem Apparat herstellen zu können. Insbesondere kann der Schlauch (graft) in einem Radius an die Pumpe herangeführt werden.

Vorzugsweise sind an den Ansatzstücken aufeinander passende ringförmige Kontaktflächen ausgebildet, die jeweils einen Durchlass durch das Ansatzstück umschließen, so dass durch Verriegeln der Ansatzstücke miteinander die Kontaktflächen aufeinandergepresst werden und automatisch eine dichtende Verbindung bilden. Insbesondere im Fall einer gegenpulsatilen Blutpumpe ist aufgrund der hohen auftretenden Drücke zum Fördern des Bluts eine gut dichtende Verbindung von Bedeutung. Hierbei ist es vorteilhaft, wenn ein radial äußerer Umfangsbereich jeder Kontaktfläche gegenüber einem dichtenden inneren Kontaktbereich um einen Winkel α von wenigen Grad und insbesondere von weniger als einem Grad zurückweicht. Dadurch berühren sich die Kontaktflächen im verriegelten Zustand nur in dem inneren Kontaktbereich, so dass in dem inneren, an den Durchlass angrenzenden Kontaktbereich die Flächenpressung erhöht wird und die Dichtwirkung verbessert ist. Dadurch, dass die höchste Flächenpressung im Bereich des blutführenden Durchlasses erzielt wird, entfällt die Notwendigkeit für ein separates Dichtungselement, zum Beispiel in Form eines Dichtrings.

Vorzugsweise wird das eine, insbesondere das mit dem Schlauch verbindbare erste Ansatzstück von einer Überwurfmutter umschlossen, die mit dem anderen, insbesondere dem mit dem Apparat verbindbaren zweiten Ansatzstück derart verriegelbar ist, dass ein Hohlraum zwischen dem anderen Ansatzstück und der Überwurfmutter gebildet ist. In diesem Holraum wird das eine, insbesondere mit dem Schlauch verbindbare erste Ansatzstück gehalten, wobei die Überwurfmutter zum Verriegeln mit dem anderen Ansatzstück ausgebildet ist. Das eine, von der Überwurfmutter umschlossene Ansatzstück wird durch die Überwurfmutter mit dem zweiten Ansatzstück verbunden und kann in dem Hohlraum in Bezug auf das andere Ansatzstück beliebig verdreht werden.

Eine Feder kann in dem Hohlraum zum Ausüben einer Kraft von der Überwurfmutter auf das eine Ansatzstück in Richtung auf das andere Ansatzstück vorgesehen sein. Im verriegelten Zustand wird das von der Überwurfmutter umschlossene Ansatzstück von der Feder gegen das andere Ansatzstück in axialer Richtung gepresst, während eine Rotation der beiden Ansatzstücke relativ zueinander ermöglicht ist. Um einen gleichmäßig verteilten Druck auf die gegeneinander gepressten Kontaktflächen im verriegelten Zustand zu erzielen, ist die Feder vorzugsweise eine Wellenfeder, die im komprimierten, verriegelten Zustand einen maximalen Druck ausübt. Die Gefahr eines Verkantens der beiden Ansatzstücke ist reduziert und die Dichtwirkung gleichmäßig über den Umfang verteilt.

Das erste, mit dem Schlauch verbindbare Ansatzstück weist vorzugsweise einen mit einer scharfen Endkante versehenen Kragen auf, auf den der Schlauch aufgeschoben wird. Die scharfe Endkante ermöglicht einen nahezu stufenlosen Übergang zwischen Schlauch und Ansatzstück, um die Gefahr einer Agglomeration von Blut zu reduzieren. Der Kragen ist an seiner Außenseite mit einer Stufe versehen, die den übergeschobenen Schlauch nach Art eines Widerhakens gegen ein Abrutschen von dem Ansatzstück sichert. Die Verbindung zwischen dem scharfkantigen ersten Ansatzstück und dem Schlauch kann bereits vor Auslieferung der Schnellkupplung mit der nötigen Sorgfalt hergestellt und versiegelt werden, um die Verletzungsgefahr für den Operateur und die Gefahr von Klafften beim Herstellen der Verbindung zu vermindern.

Vorzugsweise ist zur Verriegelung ein Bajonettverschluss zwischen Überwurfmutter und zweitem Ansatzstück vorgesehen, wobei die Überwurfmutter einen den Bajonettverschluss bedeckenden Schutzring aufweisen kann, um die Verletzungsgefahr für einen Operateur beim Herstellen der Verbindung zu vermindern oder das Eindringen von Handschuhmaterial zu reduzieren. Mit dem Bajonettverschluss kann auf einfache Weise durch Aufeinanderschieben und anschließendes Verdrehen der beiden Ansatzstücke gegeneinander die Verbindung lösbar und einfach hergestellt werden, wobei die Drehbewegung beim Schließen des Bajonettverschlusses nicht auf den Schlauch und somit nicht auf eine mögliche Nahtstelle mit einem Gefäß eines Patienten übertragen wird. Vorzugsweise sind die beiden Ansatzstücke, die Überwurfmutter und die implantierbare Feder aus Titan gefertigt, um eine stabile und leichte Schnellkupplung zu schaffen, die durch Blut nicht korrodiert. Der Schutzring ist vorzugsweise aus transparentem Kunststoff mit hoher Reibung, damit ein Operateur den Ring auch mit Operationshandschuhen gut greifen und zum Verriegeln drehen kann.

Im Folgenden wird anhand der Figuren ein Ausführungsbeispiel näher erläutert.

Es zeigen:
- Fig. 1: einen Längsschnitt durch die erfindungsgemäße Schnellkupplung mit verbundenem Schlauch,
- Fig. 2: eine seitliche Ansicht eines ersten Ansatzstücks gemäß dem Ausführungsbeispiel,
- Fig. 3: eine seitliche Ansicht eines zweiten Ansatzstücks gemäß dem Ausführungsbeispiel,
- Fig. 4: einen Längsschnitt des Bereichs IV in Figur 3 und
- Fig. 5: eine perspektivische Ansicht einer Überwurfmutter gemäß dem Ausführungsbeispiel.

Die Schnellkupplung 10 besteht aus dem ersten, mit dem Schlauch 12 verbindbaren Ansatzstück 14, dem zweiten mit dem implantierbaren Apparat verbindbaren Ansatzstück 16, der Überwurfmutter 18, der Feder 20 und dem Schutzring 23.

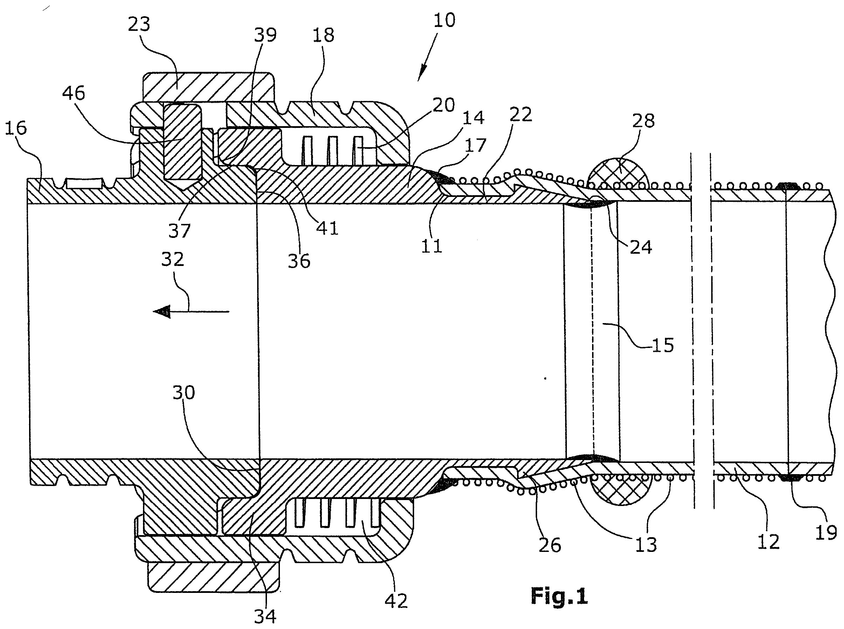

Figur 1 zeigt das erste Ansatzstück 14 mit aufgeschobenem Schlauch 12. Hierzu weist das erste Ansatzstück 14 einen abstehenden, zylindrischen Kragen 22 auf, dessen umlaufende Endkante 24 spitz zuläuft, so dass in dem Übergangsbereich zwischen Kragen 22 und Schlauch 12 keine deutlich in das Schlauchlumen hervorstehende Kante ausgebildet ist, an der Blut koagglomieren könnte. An der Außenseite ist der Kragen 22 mit einer umlaufenden Stufe 26 versehen, die einen Vorsprung in Richtung auf das zweite Ansatzstück 16 bildet und in Richtung auf die Endkante 24 schräg abfällt. Auf diese Weise wird der Schlauch 12, der dem Kragen 22 thermisch aufgeschrumpft ist, von der Stufe 26 nach Art eines Widerhakens gegen Abrutschen gesichert. Eine Nitinolspirale 13 umschließt den Schlauch 12 proximal und distal der Endkante 24 und verhindert ein Aufweiten des Schlauches 12, was zu einem Abrutschen des Schlauchs 12 von dem ersten Ansatzstück 14 und zu einem Beschädigen des Schlauchs 12 durch Reibung an der scharfen Endkante 24 führen könnte. Die Nitinolspirale 13 wird proximal auf dem Ansatzstück 14 mit einem dauerfesten und biokompatiblen Kunststoffkleber (Epoxid) 17 überdeckt. Die Stosskante 11 des Schlauches 12 wird dabei zum Ansatzstück 14 komplett verkapselt und gegen Aufweiten gesichert.

Am distalen Ende der Nitinolspirale 13 wird die letzte Wendel der Nitinolspirale 13 über die Kunststoffspirale 23 zur vorletzten Wendel der Nitinolspirale 13 zurück geführt. Die beiden Wendeln der Nitinolspirale 13 werden dort über eine kurze Distanz parallel geführt und auf Ober- und Unterseite laserverschweißt. Anschließend wird der komplette Bereich des distalen Endes der Nitinolspirale 13 über zwei Wendel mit dauerfestem und biokompatiblem Kunststoffkleber (Epoxid) 19 verkapselt. Ein spitzes Ende der Nitinolspirale 13 wird somit verhindert und eine Perforation oder Punktion des Schlauches 12 vermieden.

Der Schlauch 12 wird mit einem dauerfesten Klebstoff auf dem Ansatzstück 14 verklebt und die Endkante 24 wird mit einem dauerfesten und biokompatiblen Kunststoffkleber (Epoxid) 15, überdeckt, um die Kante 24 vollends für Blut zu eliminieren.

An dem distalen, der Endkante 24 gegenüberliegende Ende weist das erste Ansatzstück 14 eine Kontaktfläche 30 zum Kontaktieren des zweiten Ansatzstücks 16 auf. Die Kontaktfläche 30 ist ringförmig ausgebildet und umschließt einen Durchlass 32 des zylindrischen, durch die beiden Ansatzstücke 14, 16 hindurchlaufenden Lumens. Die Kontaktfläche 30 ist außen durch einen in distaler Richtung und nach außen vorstehenden zylindrischen Halterand 34 begrenzt. Der Innendurchmesser des Halterands 34 ist geringfügig größer als der Außendurchmesser am proximalen Ende des zweiten Ansatzstücks 16, so dass der Halterand 34 auf das zweite Ansatzstück 16 aufgeschoben werden kann und das proximale Ende des zweiten Ansatzstücks 16 umgreift.

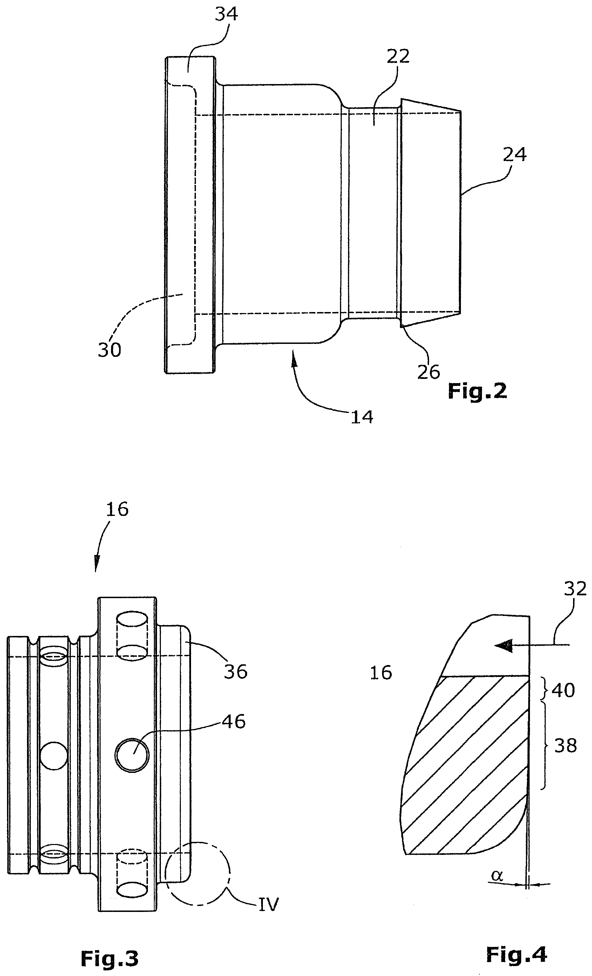

Das proximale Ende des zweiten Ansatzstücks 16 weist eine den Durchlass 32 außen umschließende, ringförmige Kontaktfläche 36 auf. Die beiden Kontaktflächen 30 und 36 sind aufeinanderpassend ausgebildet, wobei jeweils ein äußerer Umfangsbereich 38 jeder Kontaktfläche 30, 36, wie in Figur 4 dargestellt, gegenüber einem inneren Kontaktbereich 40 um einen Winkel α derart zurückweicht, dass sich die Kontaktflächen 30, 36 in dem in Figur 1 dargestellten gegeneinander gepressten Zustand nur in dem inneren Kontaktbereich 40 berühren und in dem äußeren Umfangsbereich 38 voneinander beabstandet sind. Dadurch ist die Flächenpressung in dem inneren Kontaktbereich 40 relativ hoch und eine gleichmäßig umlaufende Dichtung auf Basis eines Presssitzes geschaffen, ohne dass ein zusätzliches Dichtelement, wie z. B. ein Dichtring, benötigt wird. Die beiden Ansatzstücke 14 und 16 sind radial im Bereich 37 über einen Spielsitz angepasst, so dass beide Teile leicht zu fügen sind, der maximale radiale Versatz aber minimal ist. Entsprechend entsteht für Blut keine Stoßkante, an der sich Blutbestandteile anlagern können. Das Band der Spielpassung im Bereich 37 ist kurz und an beiden Enden mit genau angepassten Radien versehen 41 / 39, so dass zum Entlüften die Ansatzstücke 16 und 14 gegeneinander verkippt werden können. Dabei dient der Bund als Anlagekante und vereinfacht damit das Fügen. So kann in den entstehenden Schlitz/Spalt Flüssigkeit von oben appliziert werden, während die Teile ohne Einschluss von Luft gefügt werden können.

Das erste Ansatzstück 14 wird außen von der Überwurfmutter 18 umschlossen. Das proximale, in Figur 1 rechte Ende der Überwurfmutter 18 hat einen Innendurchmesser, der geringer ist als der äußere Durchmesser des Halterands 34. Dadurch wird zwischen der Überwurfmutter 18 und dem ersten Ansatzstück 14 ein Hohlraum 42 für die Feder 20 geschaffen. Die Überwurfmutter 18 kann in proximaler Richtung vollständig über das erste Ansatzstück 14 hinweggeschoben werden und wird in distaler Richtung durch den Halterand 34 gehalten. Zwischen Halterand 34 und dem proximalen Bereich mit vermindertem Innendurchmesser der Überwurfmutter 18 wird die Feder 20 bei Verschieben der Überwurfmutter 18 in distaler, in Figur 1 linker, Richtung zusammengepresst und übt auf die Überwurfmutter 18 einen Federdruck in proximaler Richtung in Bezug auf das erste Ansatzstück 14 aus. Die Feder 20 ist eine Wellenfeder zur Erzeugung eines gleichmäßig über den Umfang verteilten Federdrucks.

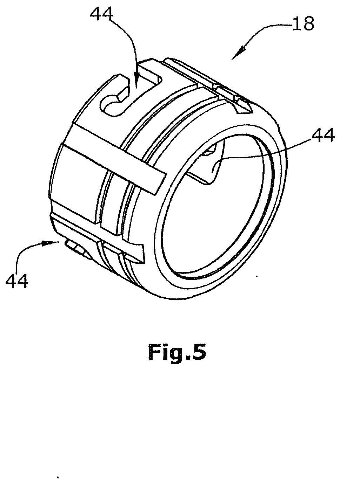

Die Überwurfmutter 18 ist, wie in Figur 5 dargestellt, an ihrem distalen, in Figur 5 linken Ende mit drei über den Umfang verteilten hakenförmigen Langlöchern 44 eines Bajonettverschlusses versehen. Die Hakenlöcher 44 greifen jeweils mit einem entsprechenden, radial von dem zweiten Ansatzstück 16 abstehenden Knopf 46 zusammen, so dass der Bajonettverschluss aus den Langlöchern 44 und den Knöpfen 46 durch eine Steck-Dreh-Bewegung der Überwurfmutter 18 relativ zu dem zweiten Ansatzstück 16 verschließbar ist und die Überwurfmutter 18 mit dem zweiten Ansatzstück 16 verriegelt. In dem in Figur 1 gezeigten verriegelten Zustand presst die Feder 20 das erste Ansatzstück 14 gegen das zweite Ansatzstück 16, wobei die inneren Kontaktbereiche 40 der Kontaktflächen 30, 36 einen dichtenden Presssitz haben.

Die Überwurfmutter 18 ist im Bereich der hakenförmigen Langlöcher 44 von einem in Figur 5 nicht dargestellten Schutzring 23 aus transparentem Kunststoff umgeben, der, wie in Figur 1 dargestellt, den Bajonettverschluss außen überdeckt. Dadurch ist vermieden, dass ein Operationshandschuh eines Operateurs beim Verriegeln der Schnellkupplung 10 in den Bajonettverschluss gerät und dort verklemmt. Zudem bildet der Kunststoffring auch mit Operationshandschuhen einen griffigen Halt. Der in Figur 1 dargestellte Ring 28 (bevorzugt aus Silikon) dient als Rückhaltering für die Überwurfmutter 18, damit diese vor dem Fügungsvorgang nicht in Richtung der Nahtstelle entweichen kann.

In dem Ausführungsbeispiel ist der mit dem Schlauch 12 zu verbindende implantierbare Apparat eine in den Figuren nicht dargestellte, gegenpulsatile Blutpumpe (CPD). Der Schlauch 12 ist ein arterieller Graft, dessen proximales, der Schnellkupplung 10 gegenüberliegendes Ende mit einer von dem Herzen aufsteigenden Arterie, typischerweise der Subclavia, fest vernäht wird, so dass der Schlauch 12 von arteriellem Blut durchströmt wird. Die Schnellkupplung ermöglicht es hierbei, die Naht zwischen Schlauch 12 und der Subclavia während einer Operation zunächst herzustellen und anschließend auf einfache Weise das der Subclavia gegenüberliegende Ende des Schlauchs 12 mit der Blutpumpe zu verbinden, ohne dass hierbei riskante Zug-, Druck- oder Torsionskräfte auf die Naht zwischen Schlauch 12 und Subclavia ausgeübt werden. Die beiden Ansatzstücke 14, 16, die Feder und die Überwurfmutter 18 bestehen aus Titan und der Schlauch 12 besteht aus einem relativ elastischen und biegsamen Material (z. B. EPTFE).

Die Verbindung zwischen erstem Ansatzstück 14 und Schlauch 12 kann bereits vorab im Labor mit der erforderlichen Sorgfalt hergestellt werden, so dass durch eine entsprechende Versiegelung die scharfe Endkante 24 keine Gefahr für einen Operateur oder für den Schlauch 12 beim Herstellen der Verbindung darstellt. Hierbei kann die Verbindung zwischen Schlauch 12 und erstem Ansatzstück 14 unter der Qualitätskontrolle des Herstellers und unabhängig von dem Operateur hergestellt werden. Der Operateur braucht bei der Implantation lediglich den Bajonettverschluss verriegeln, ohne hierbei einen Einfluss auf die Dichtheit der Verbindung zu haben.

## Patentansprüche

1. Verriegelbare Schnellkupplung (10) zum lösbaren Verbinden eines Schlauchs (12) mit einem implantierbaren Apparat, mit einem mit dem Schlauch (12) verbindbaren ersten Ansatzstück (14) und einem mit dem Apparat verbindbaren zweiten Ansatzstück (16), wobei die Ansatzstücke (14, 16) im verriegelten Zustand gegeneinander verdrehbar sind.

2. Schnellkupplung (10) nach Anspruch 1, **dadurch gekennzeichnet, dass** die Ansatzstücke (14, 16) aufeinander passende ringförmige Kontaktflächen (30, 36) aufweisen, die jeweils einen Durchlass (32) durch das Ansatzstück (14, 16) umschließen.

3. Schnellkupplung (10) nach Anspruch 2, **dadurch gekennzeichnet, dass** ein radial äußerer Umfangsbereich (38) jeder Kontaktfläche (30, 36) gegenüber einem dichtenden inneren Kontaktbereich (40) um einen Winkel (α) zurückweicht.

4. Schnellkupplung (10) nach einem der Ansprüche 1 - 3, **dadurch gekennzeichnet, dass** die Schnellkupplung (10) eine das eine Ansatzstück (14) umschließende Überwurfmutter (18) aufweist, die mit dem anderen Ansatzstück (16) derart verriegelbar ist, dass zwischen der Überwurfmutter (18) und dem anderen Ansatzstück (16) ein Hohlraum (42) gebildet ist, in dem das eine Ansatzstück (14) gehalten ist.

5. Schnellkupplung (10) nach Anspruch 4, **dadurch gekennzeichnet, dass** in dem Hohlraum (42) eine Feder (20) zum Ausüben einer Kraft von der Überwurfmutter (18) auf das eine Ansatzstück (14) in Richtung auf das andere Ansatzstück (16) vorgesehen ist.

6. Schnellkupplung (10) nach Anspruch 5, **dadurch gekennzeichnet, dass** die Feder (20) eine Wellenfeder ist.

7. Schnellkupplung (10) nach einem der Ansprüche 1 - 6, **dadurch gekennzeichnet, dass** das erste Ansatzstück (14) für den Schlauch (12) einen zylindrischen Kragen (22) mit einer scharfen Endkante (24) und mit einer Stufe (26) aufweist, die den Schlauch (12) nach Art eines Widerhakens gegen Abrutschen sichert.

8. Schnellkupplung (10) nach einem der Ansprüche 1 - 7, **dadurch gekennzeichnet, dass** die Verriegelung durch einen Bajonettverschluss (44, 46) gebildet ist.

9. Schnellkupplung (10) nach Anspruch 8, **dadurch gekennzeichnet, dass** die Schnellkupplung (10) einen den Bajonettverschluss (44, 46) bedeckenden Schutzring (23) aufweist.

10. Schnellkupplung (10) nach einem der Ansprüche 1 - 9, **dadurch gekennzeichnet, dass** die beiden Ansatzstücke (14, 16) aus Titan und alle übrigen Elemente der Schnellkupplung (10) aus Titan und/oder kunststoffimplementierbarem Edelstahl gefertigt sind.
